# FASCICULE DE BREVET EUROPEEN

(11) **EP 3 119 466 B1**
(45) Date de publication et mention de la délivrance du brevet: **06.05.2020**
(21) Numéro de dépôt: 15718513.3
(22) Date de dépôt: 16.03.2015
(51) Int. Cl.: A61M 25/02

(54) **NÉCESSAIRE POUR LE MAINTIEN ET LA PROTECTION DE CATHÉTERS PLACÉS SUR UNE ZONE DU CORPS D'UN PATIENT**
KIT ZUM HALTEN UND SCHUTZ VON IN EINEM KÖRPERBEREICH EINES PATIENTEN POSITIONIERTEN KATHETERN
KIT FOR HOLDING AND PROTECTING CATHETERS THAT ARE PLACED ON AN AREA OF THE BODY OF A PATIENT

(30) Priorité: 17.03.2014 FR 1452187
(43) Date de publication de la demande: 25.01.2017
(73) Titulaire: P & P Innovation Sarl, 13260 Cassis (FR)
(72) Inventeur: GILLI, Nathalie, F-13260 Cassis (FR)
(74) Mandataire: Barbot, Willy
(86) Numéro de dépôt international: PCT/FR2015/050627
(87) Numéro de publication internationale: WO 2015/140454

(56) Documents cités:
- US-A- 6 117 111
- US-A1- 2009 157 000
- US-A1- 2011 060 295
- US-B1- 6 280 425
- US-B1- 8 486 004

## Description

La présente invention concerne un nécessaire pour le maintien et la protection de cathéters placés sur une zone du corps d'un patient.

Un cathéter est un dispositif médical servant pour l'injection prolongée dans l'organisme de médicaments ou de solutés ou pour le retrait de fluides (tels que du sang ou du pus) de l'organisme. Le cathéter est constitué par une extrémité invasive composée par un tube de largeur et de souplesse variables, inséré dans un vaisseau ou une cavité du corps, et par une tête ou raccord, constituant la partie non invasive du cathéter et permettant la connexion avec une tubulure liée à un réservoir de récupération ou contenant un soluté ou un médicament.

Pour éviter les infections et obtenir une cicatrisation plus rapide, il est nécessaire de changer les cathéters le moins souvent possible. Ainsi, plutôt que de retirer et de réinsérer une nouvelle aiguille hypodermique chaque fois qu'un médicament doit être administré, il est souvent plus efficace et plus confortable pour le patient d'insérer une aiguille du cathéter qui est ensuite laissée en place sur la peau, parfois plusieurs jours et même plusieurs mois en fonction de leur utilisation.

Une fois posé, le cathéter doit bouger le moins possible, c'est plus particulièrement le cas lors d'anesthésie ou d'analgésie post-opératoire, mettant en œuvre des cathéters périnerveux.

Pour empêcher tout mouvement excessif de l'aiguille, cette dernière est généralement fixée au patient.

Il est connu, pour fixer un cathéter d'utiliser un ruban adhésif. Cette solution étant peu efficace, il est souvent également nécessaire de fixer les tubes flexibles s'étendant à partir du corps principal du cathéter à l'aide de bandes adhésives collées sur la peau du patient.

Un tel procédé de fixation n'est toutefois pas optimal en ce que les éléments adhésifs de fixation se détachent de sorte que le cathéter ne reste pas fixé solidement à la peau du patient, en particulier s'il est positionné au niveau d'une articulation.

Pour résoudre ce problème, il est connu de suturer le cathéter, directement à la peau du patient. Bien que cette solution soit plus sûre en termes de fixation, elle n'est évidemment pas préférée par les patients et peut entraîner d'autres complications telles que des infections.

Des dispositifs permettant le maintien facilement amovible des cathéters ont été développés et sont particulièrement utiles dans les cas où il est nécessaire d'inspecter, d'ajuster ou de remplacer ledit cathéter.

Il existe ainsi un grand nombre de documents divulguant des dispositifs et procédés suivant lesquels un moyen de retenue d'un cathéter est fixé sur un coussinet dont la surface inférieure comporte une enduction autoadhésive (protégée par une pellicule détachable) par l'intermédiaire de laquelle le coussinet peut être fixé sur la peau d'un patient après insertion de la partie invasive du cathéter dans une cavité du corps ; de tels dispositifs et procédés sont, par exemple, décrits dans les documents US-2007/0.287.963, US-6.117.163, US-2009/0.254.040.

Ces dispositifs et procédés présentent plusieurs inconvénients, par exemple, ils offrent un contact imparfait de la face autoadhésive du coussinet avec la surface de l'épiderme, compte tenu des reliefs et creux (ridules) que présente ladite surface, ce qui favorise le décollement dudit coussinet ce qui entraine une imperméabilité non assurée autour de l'entrée du cathéter, permettant le passage de l'eau et des agents infectieux en direction du site d'implantation dudit cathéter.

De plus, ces dispositifs utilisent généralement des éléments en matière plastique rigide avec des profils proéminents qui peuvent être inconfortables et douloureux pour le patient.

Enfin, un inconvénient majeur de ces dispositifs est que les cathéters sont trop exposés, de sorte qu'ils risquent de s'accrocher et d'être arrachés obligeant le patient ou les praticiens à constamment faire attention à leurs mouvements, ce qui peut être inconfortable.

Il a été proposé des dispositifs permettant de remédier à cet inconvénient, notamment à l'aide de systèmes permettant de recouvrir le cathéter, de sorte à éviter son contact avec des éléments extérieurs pouvant provoquer son arrachement.

Par exemple, le document US-2008/0.065.022 décrit (figure 3) un dispositif de fixation de cathéter comprenant, d'une part, un coussinet comportant une surface interne destinée à être appliquée sur la peau d'un patient et une surface externe, une cavité étant ménagée dans la surface interne dudit coussinet pour le logement de la partie non invasive d'un cathéter, et, d'autre part, une bande ou feuille de fixation flexible dont la partie centrale est rattachée à la face externe du coussinet, les portions d'extrémité débordantes de cette bande flexible étant munies, sur leur surface interne, d'une enduction adhésive destinée à être appliquée sur la peau d'un patient, après retrait des pellicules de protection.

Ce dispositif présente également les mêmes inconvénients que les dispositifs susmentionnés, à savoir :
- il ne permet pas de créer, autour de l'orifice d'implantation du cathéter, une barrière étanche s'opposant efficacement au passage de l'eau et des agents infectieux en direction du site d'insertion du cathéter, en raison du fait que la surface pleine enduite de matière autoadhésive du coussinet ne peut épouser étroitement les reliefs et creux (ridules) de la surface de l'épiderme ;
- il peut, pour cette même raison, se décoller facilement, de manière intempestive, avec les complications qui peuvent découler d'un mauvais positionnement du cathéter si on ne remédie pas rapidement à cette situation.

Un inconvénient commun à tous ces dispositifs, résulte du fait que leur sous face se trouve encollée lors de la mise à disposition du personnel soignant de sorte qu'ils ne permettent pas de choisir la zone cutanée d'encollage la mieux appropriée. Or ceci peut s'avérer gênant, par exemple, si le patient présente une plaie à proximité de l'emplacement du cathéter. De plus, si un cathéter doit être amené à rester en place plusieurs semaines ou plusieurs mois, les dispositifs étant toujours collés au même endroit, ils sensibilisent la peau du patient, ce qui peut créer des traumatismes cutanés ou des réactions allergiques.

Un autre inconvénient majeur de tous ces dispositifs est qu'aucun d'eux ne fournit une protection étanche des cathéters.

US 2009/0157000 décrit un système de protection de zones d'accès de cathéters comprenant un coussinet positionné autour du cathéter et maintenu en place par un adhésif.

US 6117111 décrit un outil de protection d'une blessure comprenant un drain recouvert d'une feuille de couverture maintenue en place par un adhésif.

Un objet de l'invention est de remédier aux inconvénients des dispositifs de maintien et de protection des cathéters proposés à ce jour.

Selon l'invention, ce but est atteint grâce à un nécessaire de maintien et de protection de cathéters, comme dans la revendication indépendante 1 ci-dessous, comprenant:
- d'une part, un dispositif comportant :
   un coussinet comprenant une face interne et une face externe opposée à la face interne, la face interne étant destinée à être appliquée sur la peau d'un patient autour d'un cathéter, le coussinet présentant une cavité conformée pour loger une partie non invasive (tête ou raccord) du cathéter,
   une feuille de couverture mince et souple, la feuille de couverture présentant une partie centrale solidaire du coussinet et une partie périphérique de fixation débordante et non encollée qui s'étend vers l'extérieur à partir de ladite partie centrale, autour du coussinet, la face externe du coussinet étant rapportée contre une face interne de la feuille de couverture destinée à s'appliquer sur la peau du patient, et,
- d'autre part, un conditionnement contenant un adhésif étanche à l'eau et non allergénique, ce conditionnement comportant un orifice de sortie conformé pour permettre la pose d'un cordon d'adhésif reliant la peau du patient et la face interne de la feuille de couverture, le cordon d'adhésif venant en contact avec la partie périphérique de fixation de la feuille de couverture.

L'invention est par ailleurs définie additionnellement par les revendications dépendantes 2 à 10.

L'adhésif peut être un liquide, une mousse ou de préférence un gel adhésif. Dans la suite de la description il est ainsi fait référence à un gel adhésif, bien que d'autres formes d'adhésif ne soient pas exclues.

Grâce à ce nécessaire, l'application de la partie périphérique débordante de la feuille de couverture sur le cordon de gel adhésif, permet de réaliser une jonction intime et étanche entre la peau et ladite feuille de couverture, en constituant ainsi un moyen efficace s'opposant à tout mouvement du cathéter. Le dispositif de maintien et de protection de cathéters selon l'invention procure plusieurs avantages intéressants, notamment :
- de permettre une fixation solide des cathéters. En effet, le dépôt d'un cordon continu d'adhésif entre la peau et la feuille de protection permet une fixation efficace de celle-ci. Ce cordon continu placé en liaison directe entre la peau et la feuille permet de garantir une fixation périphérique totale, sans problème de continuité ;
- de permettre une protection étanche des cathéters. En effet, le dépôt d'un cordon continu d'adhésif entre la peau et la feuille de protection permet également d'assurer une fonction de barrière parfaitement étanche. Cette barrière continue placée en liaison directe entre la peau et la feuille permet de garantir une excellente étanchéité, assurant ainsi une garantie contre les risques de souillure ou de contamination des parties protégées, par contact avec des éléments impurs, y compris avec des liquides pollués . Pour une bonne étanchéité, le cordon de gel adhésif est réalisé de préférence tout autour du coussinet, c'est-à-dire en entourant le coussinet. Le cordon de gel entoure ainsi également l'emplacement de la partie non invasive du cathéter, sans entrer en contact avec cet emplacement.
- de permettre la réalisation d'une protection « sur mesure » de cathéters dont l'emplacement est généralement très varié, par le choix d'encollage adapté à chacune des situations ;
- de permettre un accès aisé pour l'inspection du cathéter ou le remplacement du dispositif, ne nécessitant pas le retrait systématique du cathéter ;
- d'être d'utilisation facile et rapide ;
- d'être également très économique.

La partie périphérique de fixation de la feuille de couverture, c'est-à-dire la partie qui s'étend autour du coussinet, déborde de préférence largement autour du coussinet. Une large partie de fixation permet en effet de choisir avec davantage de liberté la position du cordon de gel adhésif. Il est ainsi possible, le cas échéant, de contourner une partie blessée ou altérée de la peau du patient, si une telle partie blessée ou altérée se trouve au voisinage du cathéter.

Le fait que la partie périphérique de fixation de la feuille de couverture ne soit pas encollée, lui confère une meilleure perméabilité à l'air et autorise une respiration de la peau dans la zone recouverte. Le cordon de gel réduit certes la perméabilité de la feuille de couverture dans la zone où il est appliqué. Cet effet est toutefois limité en raison du caractère local du cordon de gel adhésif. En effet le cordon de gel est appliqué de préférence de manière locale sur la partie périphérique et n'en recouvre qu'une surface limitée.

Par ailleurs, le cordon de gel est de préférence appliqué de manière à ne pas venir en contact avec le coussinet. Il est toutefois possible de mettre une mince couche de gel adhésif sur la face interne du coussinet pour faciliter son application sur la peau.

Selon une importante disposition caractéristique, l'adhésif peut présenter des propriétés d'adhérence à la peau moins fortes que ses propriétés d'adhérence à la feuille de couverture, de sorte à permettre le retrait du dispositif sans effet douloureux ni résidus d'adhésif sur la peau.

Selon un exemple d'exécution préféré, le coussinet présente une forme généralement plane.

Selon un mode d'exécution, le dispositif comporte une feuille de protection détachable appliquée contre la face externe de la feuille de couverture, cette feuille de protection présentant une souplesse inférieure à celle de ladite feuille de couverture.

Selon un exemple de réalisation, la feuille de couverture présente des dimensions permettant sa découpe, cette feuille globale étant, de préférence, exécutée dans un matériau non allergénique et facilement divisible en morceaux ou fractions de dimensions plus réduites, par exemple au moyen d'une paire de ciseaux.

Selon un exemple d'exécution, la feuille de couverture est exécutée en polyuréthane ou en polymère à base de polyuréthane.

Selon un exemple de réalisation, la feuille de couverture présente une épaisseur comprise entre 2 micromètres et 3 millimètres, et de préférence une épaisseur comprise entre 10 micromètres et 80 micromètres.

Selon un mode de réalisation, le coussinet est exécuté en tout matériau présentant la robustesse et la souplesse nécessaires pour un bon maintien du cathéter et un bon confort pour le patient tel que viscose, silicone, polypropylène, polyacrylate de sodium, cellulose.

Selon un exemple d'exécution, l'adhésif est constitué par un copolymère, de préférence à base de silicone, par exemple par une colle à base de polydiméthylsiloxane, avec groupes fonctionnels, ou à base polyacrylate ou à base acrylique.

Les buts, caractéristiques et avantages ci-dessus, et d'autres encore, ressortiront mieux de la description qui suit et des dessins annexés dans lesquels:

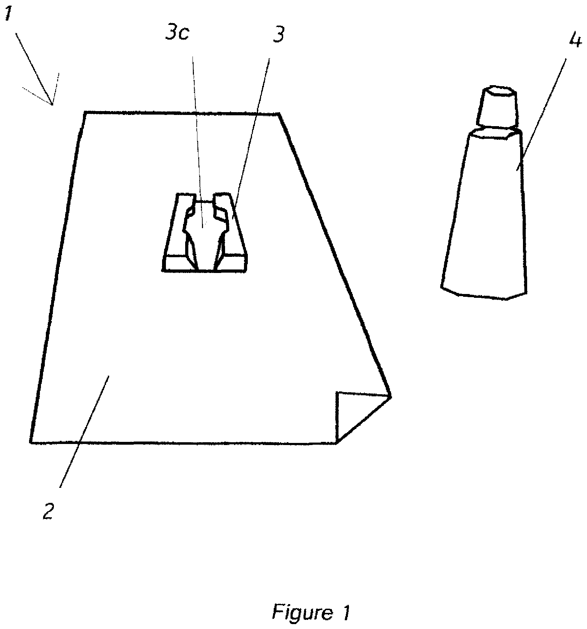

La figure 1 est une vue en perspective des éléments constituant le kit ou nécessaire selon l'invention.

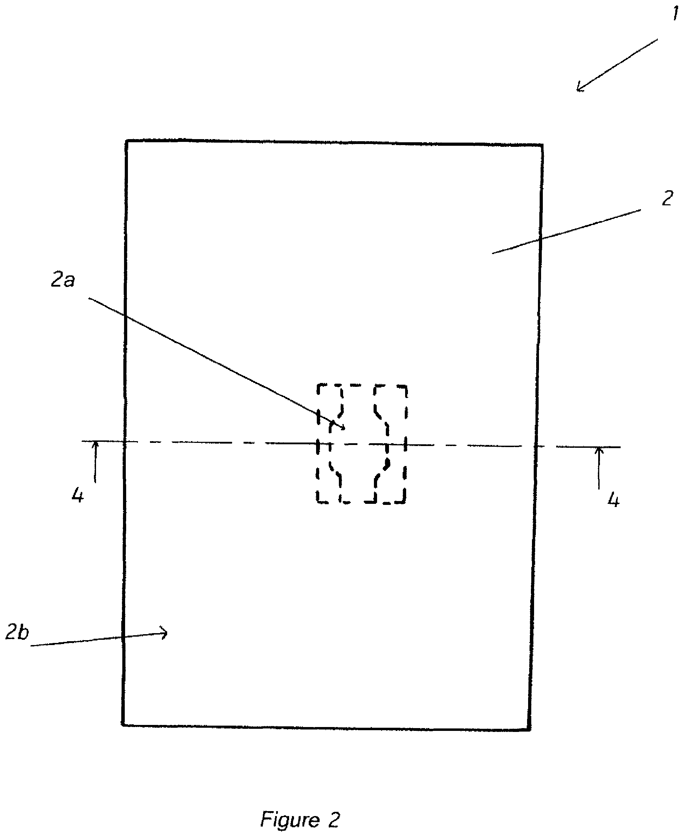

La figure 2 est une vue de dessus du dispositif de maintien.

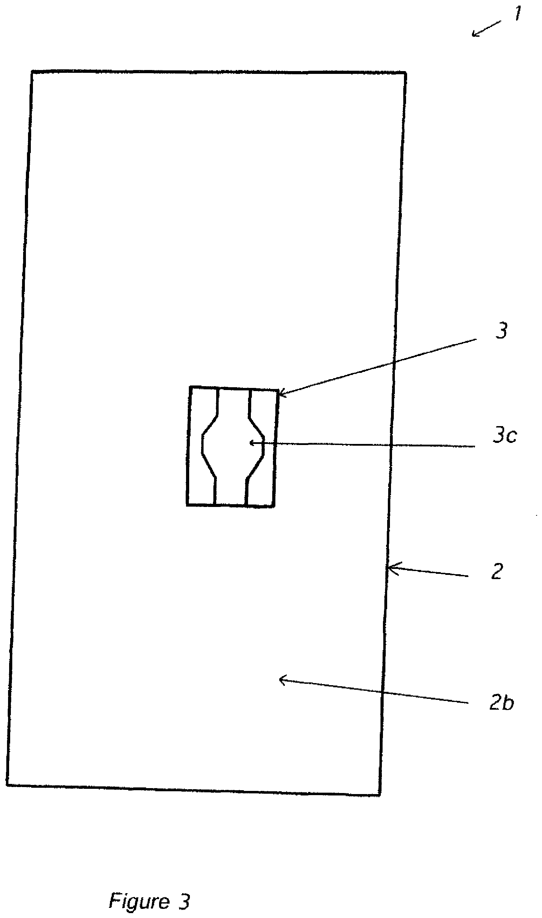

La figure 3 est une vue de dessous de celui-ci.

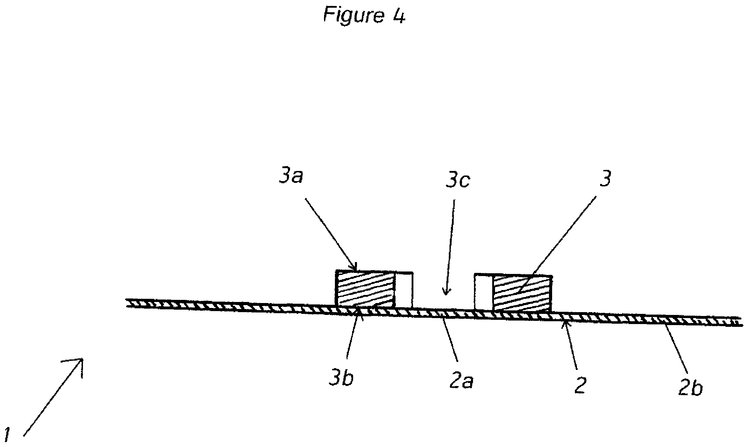

La figure 4 est une vue en coupe et à plus grande échelle, selon la ligne 4-4 de la figure 2.

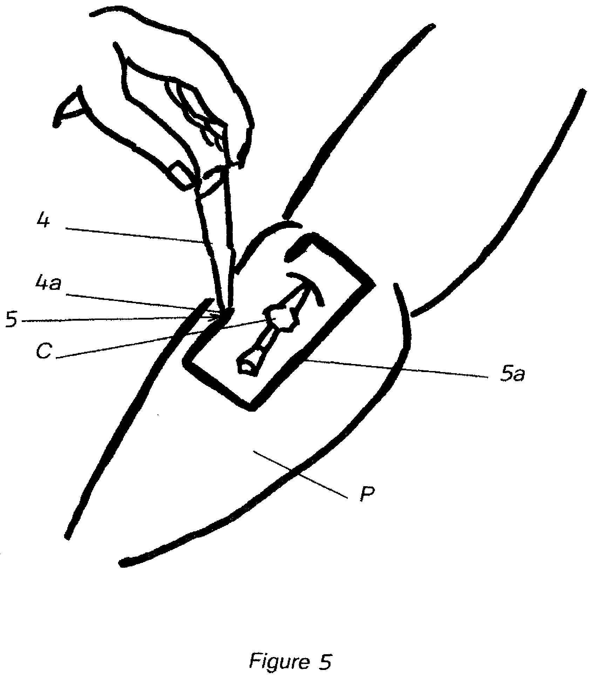

La figure 5 est une vue en perspective montrant un cathéter inséré dans un vaisseau sanguin de l'avant-bras d'une personne et la pose d'un cordon de gel autoadhésif autour de la partie non invasive du cathéter.

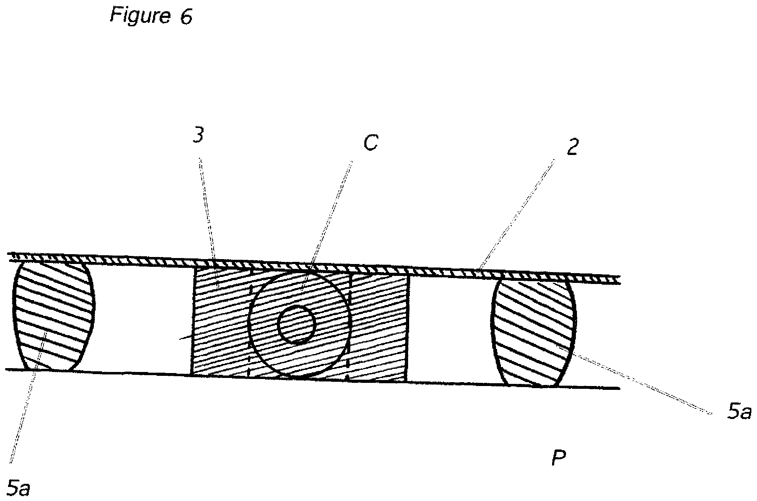

La figure 6 est une vue en coupe et à plus grande échelle montrant le dispositif de fixation et de maintien du cathéter sur l'avant-bras d'une personne.

On se reporte auxdits dessins pour décrire un exemple intéressant, quoique nullement limitatif, de réalisation du nécessaire de protection et de maintien des cathéters, selon l'invention.

Le nécessaire de protection et de maintien des cathéters selon l'invention comprend essentiellement :
- un dispositif 1 constitué d'une feuille de couverture mince et souple 2 dont la partie centrale 2a est rattachée à un coussinet 3 conformé pour loger la partie non invasive (tête ou raccord) d'un cathéter C mis en place sur une zone du corps d'un patient, et dont la portion périphérique débordante qui s'étend vers l'extérieur à partir de ladite partie centrale 2a, encore appelée bordure périphérique de fixation 2b, est non encollée ; et
- un conditionnement 4 contenant un gel adhésif 5.

La feuille de couverture 2 peut être exécutée en toute matière mince et souple et, de préférence, dans un matériau non allergénique, par exemple en polyuréthane ou en polymère à base de polyuréthane, étanche à l'eau et aux bactéries, et facilement découpable au moyen d'un instrument manuel tel qu'une paire de ciseaux. Elle présente des dimensions permettant le découpage autour du coussinet 3 appropriées à la forme et à l'emplacement des cathéters. Par exemple, elle peut présenter une longueur inférieure à 20 cm et une largeur supérieure à 4 cm. Elle peut être transparente, translucide, opaque, blanche ou teintée, voire ornée de motifs divers.

Avantageusement, le dispositif 1 comporte encore une feuille de protection détachable (non représentée) appliquée contre la face externe de la feuille de couverture 2, cette feuille de protection présentant une souplesse inférieure à celle de ladite feuille de couverture.

La face interne (face sur laquelle se trouve le coussinet 3) de la bordure périphérique 2b de la feuille de couverture 2 est destinée à s'appliquer sur la peau P du patient via un cordon de gel adhésif.

D'autre part, elle présente, de manière avantageuse, une épaisseur comprise entre 2 µm et 3 mm, et de préférence, une épaisseur comprise entre 10 µm et 80 µm.

Le coussinet 3 comporte une face interne 3a et une face externe 3b.La face interne du coussinet est destinée à être appliquée sur la peau P d'un patient. Elle peut être collée à la peau, ou, de préférence, simplement maintenue contre la peau, grâce à la feuille de couverture. Le coussinet présente une cavité 3c conformée pour loger la partie non invasive (tête ou raccord) d'un cathéter C. La face externe du coussinet est rattachée à la partie centrale 2a de la feuille de couverture 2, par tout moyen adapté connu en soi, par exemple au moyen d'une colle biocompatible. De préférence, le coussinet 3 présente une forme généralement plane.

Comme indiqué précédemment, la face externe du coussinet est rapportée contre la face interne de la feuille de couverture. Elle peut être collée ou soudée sur la feuille de couverture. Il est également possible d'utiliser une petite quantité du gel adhésif pour coller le coussinet sur la feuille de couverture, dans une présentation particulière du nécessaire de protection dans laquelle le coussinet serait fourni en étant séparé de la feuille de couverture.

Le coussinet est exécuté en tout matériau présentant la robustesse et la souplesse nécessaires pour un bon maintien du cathéter C et un bon confort pour le patient. Par exemple, il est réalisé en viscose, silicone, polypropylène, polyacrylate de sodium, cellulose, ...

Le récipient d'adhésif 4 peut être constitué par un tube compressible contenant un adhésif de consistance pâteuse 5. Bien entendu, tout autre type de récipient serait utilisable. Le récipient 4 comporte, de préférence, une tête d'extrusion ou un goulot 4a conformé, de manière connue en soi, pour permettre la distribution de l'adhésif 5 contenu dans ledit récipient sous forme d'un cordon souple 5a.

L'adhésif 5 est également non allergénique et biocompatible. Il peut être avantageusement constitué par un copolymère, de préférence à base de silicone, par exemple par une colle à base de polydiméthylsiloxane, avec groupes fonctionnels, ou à base polyacrylate ou à base acrylique. L'adhésif 5 contenu dans le récipient 4 est étanche à l'eau et possède des propriétés qui lui permettent d'adhérer à la fois à la peau P du patient et à la feuille de couverture 2, sans avoir été collé auparavant à l'une ou l'autre des deux surfaces.

De préférence et avantageusement, l'adhésif 5 présente des propriétés d'adhérence à la peau P du patient moins fortes que ses propriétés d'adhérence à la feuille de couverture 2, de sorte à permettre le retrait du dispositif 1 sans effet douloureux ni résidus d'adhésif sur la peau.

L'adhésif 5 et la feuille de couverture 2 présentent des propriétés qui en font des éléments parfaitement compatibles en adhésion pour pouvoir adhérer l'un à l'autre en situation et répondre aux contraintes. Autrement dit, l'adhésif 5 et la feuille de couverture 2 sont réalisés dans des matériaux choisis pour leurs propriétés de telle sorte qu'ils peuvent adhérer l'un à l'autre de manière à assurer l'étanchéité nécessaire.

Selon le mode de mise en œuvre du nécessaire de maintien et de protection selon l'invention, le cathéter est inséré dans une lumière d'une cavité du corps ou d'un vaisseau sanguin d'un patient, à l'endroit désiré en fonction de son utilisation ; un cordon 5a d'adhésif souple étanche à l'eau et non allergénique, et présentant en outre des propriétés lui permettant d'adhérer à la fois à la peau et à la feuille de protection, est déposé sur la zone la mieux appropriée de la peau P du patient, autour de l'emplacement de la partie non invasive (tête ou raccord) du cathéter C à protéger et à maintenir, le gel adhésif 5 s'engouffre dans les ridules de la peau réalisant ainsi une barrière étanche contre les risques de souillure ou de contamination par contact avec des éléments impurs, y compris avec des liquides pollués.

Le dispositif de maintien 1 est ensuite placé sur le patient en couverture du cathéter, de sorte, d'une part, à faire correspondre la cavité 3c de logement du cathéter C avec la partie non invasive dudit cathéter C en réalisant ainsi un maintien intime de ce dernier et, d'autre part, que la bordure périphérique débordante 2b de la feuille de couverture 2, mince, souple, étanche à l'eau et non encollée, de préférence réalisée dans un matériau non allergénique, vienne s'appliquer sur le cordon périphérique d'adhésif 5a, en réalisant ainsi une jonction intime entre la peau et la feuille de couverture individuelle 2 et l'isolement étanche du cathéter C, offrant ainsi une jonction intime et étanche entre la peau P et ladite feuille de couverture 2, en constituant un moyen efficace s'opposant à tout mouvement du cathéter C.

La portion de bordure 2b s'étendant au-delà du cordon périphérique de gel adhésif 5a par rapport au cathéter C peut être découpée aux dimensions et formes souhaitables, en fonction de la taille, de la forme et de l'emplacement du cathéter C.

Le dispositif de protection et de maintien selon l'invention ainsi appliqué sur la peau d'un patient peut rester en place plusieurs jours.

Lorsque le dispositif 1 doit être remplacé, par exemple pour l'inspection du cathéter, la feuille de couverture est enlevée en tirant sur cette dernière. Le couple de liaison entre l'adhésif 5 et la peau P du patient étant moins important que le couple de liaison entre l'adhésif 5 et la feuille de couverture 2, le dispositif 1 peut être retiré sans douleur ni effet traumatisant pour le patient, et sans laisser de résidus d'adhésif sur la peau de ce dernier.

Lors de la pose d'un nouveau dispositif 1, un nouveau cordon 5a de gel adhésif 5 est déposé sur la zone la mieux appropriée de la peau P du patient, autour de l'emplacement de la partie non invasive (tête ou raccord) du cathéter C, mais en variant légèrement la zone d'encollage par rapport à la zone d'encollage précédente.

Les zones d'encollage peuvent ainsi être alternées à chaque remplacement du dispositif 1 afin d'éviter les risques allergiques ou d'éventuels traumatismes cutanés qui pourraient survenir en disposant le gel adhésif trop longtemps sur une même surface de peau.

## Revendications

1. Nécessaire pour la protection et le maintien de cathéters, **caractérisé en ce qu'**il comprend :
- d'une part,
un coussinet (3) comprenant une face interne (3a) et une face externe (3b), opposée à la face interne, la face interne du coussinet étant destinée à être appliquée sur la peau (P) d'un patient autour d'un cathéter (C), le coussinet présentant une cavité (3c) conformée pour loger une partie non invasive (tête ou raccord) du cathéter,
une feuille de couverture mince et souple, la feuille de couverture présentant une partie centrale (2a) solidaire du coussinet et une partie périphérique de fixation (2b) débordante et non encollée qui s'étend vers l'extérieur à partir de ladite partie centrale, autour du coussinet, la face externe du coussinet (3b) étant rapportée contre une face interne de la feuille de couverture destinée à s'appliquer sur la peau du patient, et,
- d'autre part,
un conditionnement (4) contenant un adhésif (5) étanche à l'eau et non allergénique, ce conditionnement comportant un orifice de sortie conformé pour permettre la pose d'un cordon (5a) d'adhésif reliant localement la peau du patient et la face interne de la feuille de couverture, le cordon (5a) d'adhésif venant en contact avec la partie périphérique de fixation de la feuille de couverture.

2. Nécessaire pour la protection et le maintien de cathéters selon la revendication 1, **caractérisé en ce que** l'adhésif (5) présente des propriétés d'adhérence à la peau (P) moins fortes que ses propriétés d'adhérence à la feuille de couverture (2), de sorte à permettre le retrait du dispositif (1) sans effet douloureux ou traumatisant pour le patient, ni résidus d'adhésif sur la peau de ce dernier.

3. Nécessaire pour la protection et le maintien de cathéters selon l'une des revendications 1 ou 2, **caractérisé en ce que** l'adhésif (5) et la feuille de couverture (2) présentent des propriétés qui en font des éléments parfaitement compatibles en adhésion pour pouvoir adhérer l'un à l'autre en situation et répondre aux contraintes, c'est-à-dire que l'adhésif (5) et la feuille de couverture (2) sont réalisés dans des matériaux choisis pour leurs propriétés de telle sorte qu'ils peuvent adhérer l'un à l'autre de manière à assurer l'étanchéité nécessaire.

4. Nécessaire pour la protection et le maintien de cathéters selon l'une quelconque des revendications 1 à 3, **caractérisé en ce que** la feuille de couverture (2) est exécutée en polyuréthane, ou en polymère à base de polyuréthane.

5. Nécessaire pour la protection et le maintien de cathéters selon l'une quelconque des revendications 1 à 4, **caractérisé en ce que** le coussinet (3) présente une forme généralement plane.

6. Nécessaire pour la protection et le maintien de cathéters selon l'une quelconque des revendications 1 à 5, **caractérisé en ce que** le dispositif (1) comporte une feuille de protection détachable appliquée contre la face externe de la feuille de couverture (2), cette feuille de protection présentant une souplesse inférieure à celle de ladite feuille de couverture.

7. Nécessaire pour la protection et le maintien de cathéters selon l'une quelconque des revendications 1 à 6, **caractérisé en ce que** la feuille de couverture (2) présente des dimensions permettant sa découpe, cette feuille globale étant, de préférence, exécutée dans un matériau non allergénique et facilement divisible en morceaux ou fractions de dimensions plus réduites, par exemple au moyen d'une paire de ciseaux.

8. Nécessaire pour la protection et le maintien de cathéters selon l'une quelconque des revendications 1 à 7, **caractérisé en ce que** la feuille de couverture (2) présente une épaisseur comprise entre 2 µm et 3 mm et, de préférence une épaisseur comprise entre 10 µm et 80 µm.

9. Nécessaire pour la protection et le maintien de cathéters selon l'une quelconque des revendications 1 à 8, **caractérisé en ce que** le coussinet (3) est exécuté en tout matériau présentant la robustesse et la souplesse nécessaires pour un bon maintien du cathéter et un bon confort pour le patient, tel que viscose, silicone, polypropylène, polyacrylate de sodium, cellulose.

10. Nécessaire pour la protection et le maintien de cathéters selon l'une quelconque des revendications 1 à 9, **caractérisé en ce que** l'adhésif (5) est constitué par un copolymère à base de silicone, ou à base polyacrylate ou à base acrylique.

## Patentansprüche

1. Zubehör zum Schutz und zum Halten von Kathetern, **dadurch gekennzeichnet, dass** es umfasst:
- einerseits
ein Kissen (3) mit einer Innenseite (3a) und einer Außenseite (3b), der Innenseite gegenüber, wobei die Innenseite des Kissens dazu bestimmt ist, an die Haut (P) eines Patienten um einen Katheter (C) herum angelegt zu werden, wobei das Kissen eine Vertiefung (3c) aufweist, dafür geformt, einen nicht invasiven Teil (Kopf oder Anschluss) des Katheters aufzunehmen,
eine dünne und weiche Deckfolie, wobei die Deckfolie einen Zentralbereich (2a) aufweist, der mit dem Kissen fest verbunden ist, und einen peripheren Befestigungsbereich (2b), der darüber hinaus ragt und nicht mit Klebstoff versehen ist, der sich vom genannten Zentralbereich auswärts erstreckt, um das Kissen herum, wobei die Außenseite des Kissens (3b) an eine Innenseite der Deckfolie angesetzt ist, die dazu bestimmt ist, sich an die Haut des Patienten anzulegen, und
- andererseits
eine Verpackung (4), einen wasserbeständigen und nicht allergenen Klebstoff (5) enthaltend, wobei diese Verpackung eine Austrittsöffnung aufweist, dafür geformt, das Auftragen eines Klebstoffwulstes (5a) zu erlauben, der die Haut des Patienten und die Innenseite der Deckfolie örtlich verbindet, wobei der Klebstoffwulst (5a) mit dem peripheren Befestigungsbereich der Deckfolie in Berührung kommt.

2. Zubehör zum Schutz und zum Halten von Kathetern nach Patentanspruch 1, **dadurch gekennzeichnet, dass** der Klebstoff (5) weniger starkes Haftvermögen an der Haut (P), als an der Deckfolie (2) aufweist, derart, dass er das Abnehmen der Vorrichtung (1) erlaubt, ohne dem Patienten Schmerzen oder Verletzungen zu verursachen oder Klebstoffreste auf dessen Haut zu hinterlassen.

3. Zubehör zum Schutz und zum Halten von Kathetern nach Patentanspruch 1 oder 2, **dadurch gekennzeichnet, dass** der Klebstoff (5) und die Deckfolie (2) Eigenschaften aufweisen, die sie zu vollkommen haftungskompatiblen Teilen machen, um in Stellung aneinander zu haften und auf die Beanspruchungen zu reagieren, d.h. dass der Klebstoff (5) und die Deckfolie (2) aus Werkstoffen bestehen, die für ihre Eigenschaften derart ausgewählt sind, dass sie in der Weise aneinander haften können, dass die erforderliche Dichtigkeit sichergestellt wird.

4. Zubehör zum Schutz und zum Halten von Kathetern nach irgendeinem der Patentansprüche 1 bis 3, **dadurch gekennzeichnet, dass** die Deckfolie (2) aus Polyurethan besteht oder aus Polymer auf Polyurethanbasis.

5. Zubehör zum Schutz und zum Halten von Kathetern nach irgendeinem der Patentansprüche 1 bis 4, **dadurch gekennzeichnet, dass** das Kissen eine im Wesentlichen ebene Form aufweist.

6. Zubehör zum Schutz und zum Halten von Kathetern nach irgendeinem der Patentansprüche 1 bis 5, **dadurch gekennzeichnet, dass** die Vorrichtung (1) eine abnehmbare Schutzfolie aufweist, die an der Außenseite der Deckfolie (2) anliegt, wobei diese Schutzfolie eine Verformbarkeit aufweist, die geringer ist, als die der Deckfolie.

7. Zubehör zum Schutz und zum Halten von Kathetern nach irgendeinem der Patentansprüche 1 bis 6, **dadurch gekennzeichnet, dass** die Deckfolie (2) Abmessungen aufweist, die ihr Schneiden ermöglicht, wobei diese globale Folie vorzugsweise aus nicht allergenem und leicht, beispielsweise mit Hilfe einer Schere, in Stücke oder kleinere Fragmente zerlegbarem Werkstoff besteht.

8. Zubehör zum Schutz und zum Halten von Kathetern nach irgendeinem der Patentansprüche 1 bis 7, **dadurch gekennzeichnet, dass** die Deckfolie (2) eine Dicke aufweist, die zwischen 2 µm und 3 mm liegt und vorzugsweise eine Dicke, die zwischen 10 µm und 80 µm liegt.

9. Zubehör zum Schutz und zum Halten von Kathetern nach irgendeinem der Patentansprüche 1 bis 8, **dadurch gekennzeichnet, dass** das Kissen (3) aus einem beliebigen Werkstoff besteht, der die erforderliche Widerstandsfähigkeit und Verformbarkeit für ein gutes Halten des Katheters und einen hohen Komfort des Patienten aufweist, wie etwa Viskose, Silikon, Polypropylen, Natriumpolyacrylat, Zellulose.

10. Zubehör zum Schutz und zum Halten von Kathetern nach irgendeinem der Patentansprüche 1 bis 9, **dadurch gekennzeichnet, dass** der Klebstoff (5) aus einem Kopolymer auf der Basis von Silikon besteht oder auf Polyacrylatbasis oder auf Acrylbasis.

## Claims

1. Kit for the protection and maintenance of catheters, **characterised in that** it comprises:
- on the one hand,
a pad (3) including an internal face (3a) and an external face (3b), opposite to the internal face, the internal face of the pad is intended to be applied onto the skin (P) of a patient around a catheter (C), the pad with a cavity (3c) shaped to accommodate a non-invasive section (head or fitting) of catheter,
a thin and flexible covering film, the covering film with a central section (2a) integral of the pad and surrounding overflowing section attached (2b) and not glued, that extends outwards from said central section, around the pad, the external face of the pad (3b) being applied against an internal face of the covering film intended to be applied on the skin of the patient, and,
- on the other hand,
a package (4) containing an adhesive (5) which is waterproof and non-allergenic, this package with an outlet port shaped to allow positioning of a bead (5a) of the adhesive connecting locally to the skin of the patient and the internal face of the covering film, the bead (5a) of the adhesive coming into contact with the surrounding section of attached covering film.

2. Kit for the protection and maintenance of catheters according to claim 1, **characterised in that** the adhesive (5) presents properties of adhesion to the skin (P), less strong than its properties of adhesion to the covering film (2), to enable the removal of the device (1) without painful or traumatic effect for the patient, or residues of adhesive on the patient's skin.

3. Kit for the protection and maintenance of catheters according to any of claims 1 or 2, **characterised in that** the adhesive (5) and the covering film (2) present properties that make the elements perfectly compatible in adhesion to be able to adhere to one another in position and to respond to the constraints, that is to say that the adhesive (5) and the covering film (2) are made in materials chosen for their properties so that they can adhere to one another in such a way as to ensure the necessary sealing.

4. Kit for the protection and maintenance of catheters according to any one of claims 1 to 3, **characterised in that** the covering film (2) is made of polyurethane, or polymer based from polyurethane.

5. Kit for the protection and maintenance of catheters according to any one of claims 1 to 4, **characterised in that** the pad (3) presents a shape generally flat.

6. Kit for the protection and maintenance of catheters according to any one of claims 1 to 5, **characterised in that** the device (1) includes a film of detachable protection applied against the external face of the covering film (2), this protection film presenting a flexibility lower than that of said covering film.

7. Kit for the protection and maintenance of catheters according to any one of claims 1-6, **characterised in that** the covering film (2) presents dimensions allowing cutting, overall this sheet being, preferably, executed in a non-allergenic material and easily divided into pieces or fragments of smaller dimensions, for example by means of a pair of scissors.

8. Kit for the protection and maintenance of catheters according to any one of claims 1 to 7, **characterised in that** the covering film (2) presents a thickness between 2 µm and 3 mm and, preferably a thickness of between 10 .µm and 80 µm.

9. Kit for the protection and maintenance of catheters according to any one of claims 1 to 8, **characterised in that** the pad (3) is executed in any material with the robustness and flexibility necessary for firm holding of the catheter and good comfort for the patient, such as viscose, silicone, polypropylene, sodium polyacrylate, cellulose.

10. Kit for the protection and maintenance of catheters according to any one of claims 1 to 9, **characterised in that** the adhesive (5) consists of a copolymer based of silicone, or polyacrylate or acrylic.
